(19) Europäisches Patentamt
European Patent Office
Office européen des brevets

(11) Veröffentlichungsnummer: **0 052 319**
**A1**

(12) **EUROPÄISCHE PATENTANMELDUNG**

(21) Anmeldenummer: 81109563.7

(22) Anmeldetag: 07.11.81

(51) Int. Cl.[3]: C 07 C 143/70
C 25 B 3/08, B 01 F 17/00

(30) Priorität: 18.11.80 DE 3043427

(43) Veröffentlichungstag der Anmeldung:
26.05.82 Patentblatt 82/21

(84) Benannte Vertragsstaaten:
BE DE FR GB IT NL

(71) Anmelder: BAYER AG
Zentralbereich Patente, Marken und Lizenzen
D-5090 Leverkusen 1, Bayerwerk(DE)

(72) Erfinder: Geisler, Klaus, Dr.
Petrusstrasse 7
D-5300 Bonn 3(DE)

(54) **Perfluorierte Sulfonsäurefluoride sowie Verfahren zu deren Herstellung.**

(57) Die Erfindung betrifft perfluorierte Sulfonsäurefluoride der allgemeinen Formel

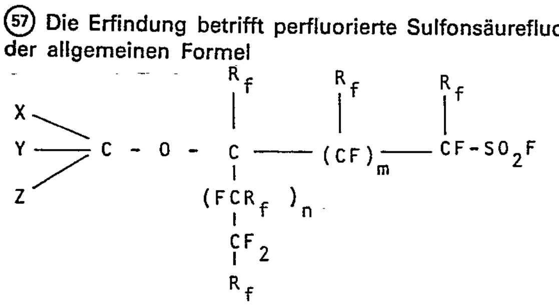

(1)

in denen X, Y und Z unabhängig voneinander für F, einen Perfluoralkylrest mit 1 - 8 C-Atomen, in dem gegebenenfalls eine oder mehrere $CF_2$-Gruppen durch Sauerstoff ersetzt sein können, wobei die Sauerstoffatome durch mindestens eine $CF_2$-Gruppe voneinander getrennt sind, einen gegebenenfalls perfluoralkylsubstituierten Cycloperfluoralkylrest mit 5 - 12 C-Atomen, in dem eine oder mehrere $CF_2$-Gruppen durch Sauerstoff ersetzt sein können.

X und Y gemeinsam für einen gegebenenfalls perfluoralkylsubstituierten Cycloperfluoralkylrest, in dem eine oder mehrere $CF_2$-Gruppen durch Sauerstoff substituiert sein können, $R_f$ unabhängig für F oder einen Perfluoralkylrest mit 1 - 3 C-Atomen steht, und n und m für die Zahlen 0 oder 1 stehen. Die Erfindung betrifft ferner ein Verfahren zur Herstellung der genannten perfluorierten Sulfonsäurefluoride.

EP 0 052 319 A1

BAYER AKTIENGESELLSCHAFT 5090 Leverkusen, Bayerwerk

Zentralbereich
Patente, Marken und Lizenzen Br/Kü-c

# Perfluorierte Sulfonsäurefluoride sowie Verfahren zu deren Herstellung

Die Erfindung betrifft perfluorierte Sulfonsäurefluoride der allgemeinen Formel

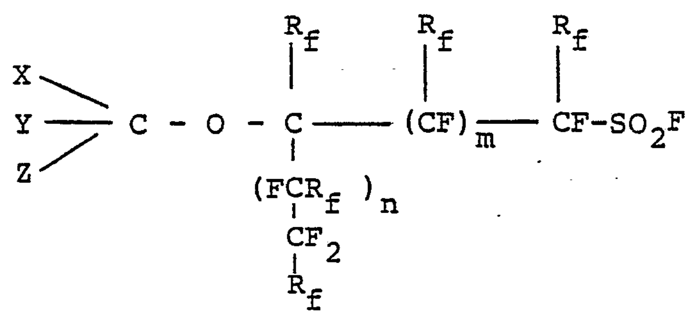

(I)

in denen X, Y und Z unabhängig voneinander für F, einen Perfluoralkylrest mit 1 - 8 C-Atomen, in dem gegebenenfalls eine oder mehrere $CF_2$-Gruppen durch Sauerstoff ersetzt sein können, wobei die Sauerstoffatome durch mindestens eine $CF_2$-Gruppe voneinander getrennt sind, einen gegebenenfalls perfluoralkylsubstituierten Cycloperfluoralkylrest mit 5 - 12 C-Atomen, in dem eine oder mehrere $CF_2$-Gruppen durch Sauerstoff ersetzt sein können,

X und Y gemeinsam für einen gegebenenfalls perfluoralkylsubstituierten Cycloperfluoralkylrest, in dem

eine oder mehrere $CF_2$-Gruppen durch Sauerstoff substituiert sein können, $R_f$ unabhängig für F oder einen Perfluoralkylrest mit 1 - 3 C-Atomen steht, und n und m für die Zahlen 0 oder 1 stehen. Die Erfindung betrifft ferner ein Verfahren zur Herstellung der genannten perfluorierten Sulfonsäurefluoride.

Bei der herkömmlichen Elektrofluorierung von Alkylsulfonsäurehalogeniden zu Perfluoralkylsulfonsäurefluoriden werden mit steigender Kettenlänge niedrigere Ausbeuten erhalten, die ihre Ursache in Spaltungen oder Umlagerungen des Kohlenstoffgerüsts sowie Polymerisation unter Schlammbildung haben. Besonders bei den technisch interessanten längerkettigen Perfluoralkylsulfonylfluoriden führt das Verfahren nur zu unbefriedigenden Ausbeuten (vgl. z.B.: J. Chem. Soc., 1957, 2640). Hinzu kommt, daß die Ausgangsverbindungen, Alkyl-1-sulfonsäurefluoride oder -chloride, nur durch mehrstufige, aufwendige und zu zahlreichen nicht nutzbaren Nebenprodukten führende Verfahren zugänglich sind. So führt z.B. die Synthese von $C_8H_{17}SO_2F$ zu mindestens der 1,7-fachen Menge an NaCl, das entweder über das Abwasser abgeführt oder mit aufwendigen Reinigungsverfahren beseitigt werden muß.

Es liegt daher der Erfindung die Aufgabe zugrunde, in einem verbesserten Verfahren neue, mindestens ein Sauerstoffatom in Etherfunktion enthaltende perfluorierte Sulfonsäurefluoride zu synthetisieren, nach dem die gewünschten Verbindungen in vergleichbar mit den bisherigen Verfahren guter Ausbeute aus leicht zugänglichen Ausgangsverbindungen erhalten werden.

Die Lösung dieser Aufgabe wurde überraschenderweise in dem Weg gefunden, die nach DE-PS 682 079 leicht zugänglichen Additionsprodukte von primären, sekundären und tertiären Alkoholen an $\Delta$ 3-Dihydrothiophen-1,1-dioxide (wie z.B. Sulfolen) der allgemeinen Formel

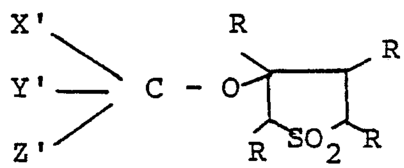

II,

in der X', Y' und Z' unabhängig voneinander für H, einen Alkylrest mit 1 - 8 C-Atomen, in dem eine oder mehrere $CH_2$-Gruppen durch Sauerstoff substituiert sein können, wobei die Sauerstoffatome durch mindestens eine $CH_2$-Gruppe voneinander getrennt sind, einen gegebenenfalls alkylsubstituierten Cycloalkylrest mit 5 - 12 C-Atomen, in dem eine oder merhere der $CH_2$-Gruppen durch Sauerstoff ersetzt sein können,

X' und Y' gemeinsam für einen gegebenenfalls alkylsubstituierten Cycloalkylrest, in dem eine oder

mehrere $CH_2$-Gruppen durch Sauerstoff substituiert sein können und R unabhängig für H oder einen Alkylrest mit 1 bis 3 C-Atomen steht, der elektrochemischen Fluorierung zu unterwerfen.

Nach GB-PS 1 099 240 und BE-PS 747 306 ist die elektrochemische Fluorierung von cyclischen Sulfonen zu Perfluoralkylsulfonylfluoriden an sich bekannt. Völlig überraschend ist jedoch, daß auch diese substituierten Sulfone ohne Spaltung der Etherbindung zu perfluoralkylsubstituierten offenkettigen Sulfonylfluoriden elektrofluoriert werden können, da andererseits eine der Schwefel-Kohlenstoff-Bindung fluorierend gespalten wird.

Die bevorzugte Klasse an Ausgangsverbindungen der allgemeinen Formel II sind diejenigen, in denen X', Y' und Z' für H, X' und Y' für einen kurzkettigen Alkylrest mit 1 - 6 C-Atomen, in denen gegebenenfalls maximal zwei $CH_2$-Gruppen durch Sauerstoff substituiert sind oder X' und Y' gemeinsam für einen gegebenenfalls alkylsubstituierten Cycloalkylrest mit 5 - 8 Kohlenstoffatomen, wobei einzelne $CH_2$-Gruppen durch Sauestoff ersetzt sein können, sowie R unabhängig für H oder eine Methylgruppe stehen.

Beispiele für die bei dem erfindungsgemäßen Verfahren benutzten Ausgangsverbindungen sind 3-Methoxy-

tetrahydrothiophen-1,1-dioxid, 3-Ethoxy-tetrahydrothiophen-1,1-dioxid, 3-n-Propoxy-tetrahydrothiophen-1,1-dioxid, 3-i-Propoxy-tetrahydrothiophen-1,1-dioxid, 3-n-Butoxy-tetrahydrothiophen-1,1-dioxid, 3-sec-Butoxy-tetrahydrothiophen-1,1-dioxid, 3-tert.-Butoxy-tetrahydrothiophen-1,1-dioxid, 3-(2-Ethyl)-hexoxy-tetrahydrothiophen-1,1-dioxid, 3-(2-Methoxy)-ethoxy-tetrahydrothiophen-1,1-dioxid-3-(2-Ethoxy)-ethoxy-tetrahydrothiophen-1,1-dioxid, 3-Cyclohexoxy-tetrahydrothiophen-1,1-dioxid, 3-(4-Methyl)-cyclohexoxy-tetrahydrothiophen-1,1-dioxid, 3-(4-Methoxy)-cyclohexoxy-tetrahydrothiophen-1,1-dioxid, 3-Methyl-4-methoxy-tetrahydrothiophen-1,1-dioxid, 2-Ethyl-3-methoxy-tetrahydrothiophen-1,1-dioxid, 3-Methyl-3-methoxy-tetrahydrothiophen-1,1-dioxid und 2-Methyl-3-methoxy-tetrahydrothiophen-1,1-dioxid.

Das Verfahren zur elektrochemischen Fluorierung selbst wird unter anderen in den US-Patentschriften 2 717 871 und 2 519 983, in Houben-Weyl, Methoden der organischen Chemie, Bd. C, T. 3, S. 42 - 50 und in den DE-OS 2 442 106 sowie DE-OS 2 725 211 näher beschrieben. Das erfindungsgemäße Verfahren wird bei einer Substratkonzentration von 0,2 bis 10 Gewichtsprozent in Fluorwasserstoff, einer Elektrolysespannung zwischen 4,5 und 15 V, einer Stromdichte von 4 - 20 $mA/cm^2$ wirksamer Anodenfläche sowie einer Temperatur zwischen -20°C und 50°C, bevorzugt zwischen 0 und 15°C, durchgeführt.

Die Ausgangsverbindungen der allgemeinen Formel II werden zu Beginn der Elektrolyse in Fluorwasserstoff in einer Menge von 0,2 bis 10 Gewichtsprozent gelöst. Die resultierenden Lösungen leiten den elektrischen Strom. Während der Elektrolyse werden Fluorwasserstoff und organisches Substrat diskontinuierlich oder kontinuierlich je nach Verbrauch ersetzt. Der kathodisch gebildete Wasserstoff wird an aufsteigenden, mit Kühlsole beschickten Rückflußkühlern weitgehend von mitgeführtem Fluorwasserstoff befreit und dieser in die Elektrolytzelle zurückgeführt. Die Perfluorrohprodukte sammeln sich als spezifisch schwere Flüssigkeiten an einer tiefliegenden Stelle der Elektrolyseapparatur und werden von Zeit zu Zeit ausgeschleust.

Durch die Erfindung wird erreicht, daß perfluorierte Sulfonsäurefluoride der allgemeinen Formel I

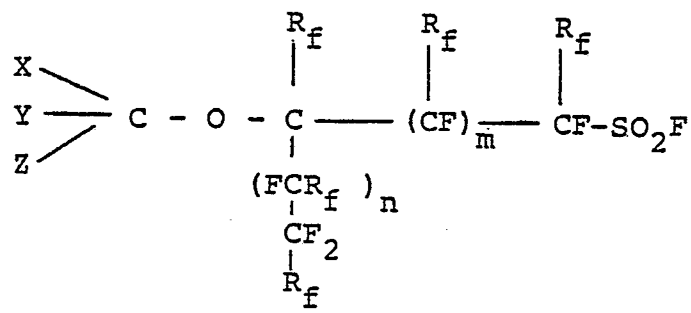

wobei X, Y, Z, $R_f$ sowie n und m die vorstehend genannten Bedeutungen haben, in, vergleichsweise zur Herstellung

von Perfluoralkylsulfonylfluoriden gleicher Kettenlänge, guten Ausbeuten entstehen. Beispiele für erfindungsgemäß hergestellte Verbindungen sind 4-Oxa-3-trifluormethyl-perfluorpentyl-1-sulfofluorid, 3-Oxa-2-pentafluorethyl-perfluorbutyl-1-sulfofluorid, 4-Oxa-3-trifluormethyl-perfluorhexyl-1-sulfofluorid, 3-Oxa-2-pentafluorethyl-perfluorpentyl-1-sulfofluorid, 4-Oxa-3,5-bis-trifluormethyl-perfluorhexyl-1-sulfofluorid, 3-Oxa-2-pentafluorethyl-4-trifluormethyl-perfluorpentyl-1-sulfofluorid, 4-Oxa-3-trifluormethyl-6-pentafluorethyl-perfluordecyl-1-sulfofluorid, 3-Oxa-2,5-bis-pentafluorethyl-perfluornonyl-1-sulfofluorid, 4,7-Dioxa-3-trifluormethyl-perfluoroctyl-1-sulfofluorid, 3,6-Dioxa-2-pentafluorethyl-perfluorheptyl-1-sulfofluorid, 4-Oxa-3-trifluormethyl-4-perfluorcyclohexyl-perfluorbutyl-1-sulfofluorid, 3-Oxa-2-pentafluorethyl-3-perfluorcyclohexyl-perfluorpropyl-1-sulfofluorid, 4-Oxa-3-pentafluorethyl-perfluorpentyl-1-sulfofluorid und 3-Oxa-1-trifluormethyl-2-pentafluorethyl-perfluorbutyl-1-sulfofluorid.

Nach $^{19}F$-NMR- und gaschromatographischen Untersuchungen werden die isomeren Verbindungen I mit m=1, n=0 und m=0, n=1 aus ein und derselben Ausgangsverbindung II etwa in einem Gewichtsverhältnis wie 1 : 4 erhalten.

Die perfluorierten Sulfonsäurefluoride der allgemeinen Formel I sind für zahlreiche Zwecke nutzbar. So lassen sie sich gemäß DE-OS 1 929 665 in das Tetraethylammoniumsalz der jeweiligen Oxaperfluorsulfonsäure überführen. Diese verleihen Wasser

in sehr geringen Einsatzkonzentrationen eine niedrige Oberflächenspannung und lassen sich damit als Netz- und Verlaufsmittel nutzen. Durch alkalische oder ammonialkalische Hydrolyse erhält man die analogen Alkali- und Ammoniumsalze, die ebenfalls als Tenside eingesetzt werden. Die Umsetzung mit wasserfreiem Ammoniak und primären Aminen führt zu Oxaperfluoralkylsulfonamiden, die als Ausgangsverbindungen für die schmutzabweisende sowie hydro- und oleophobe Ausrüstung von Leder-, Papier- und Textiloberflächen dienen.

Die folgenden Beispiele erläutern die Erfindung, ohne sie jedoch auf diese zu begrenzen.

## Beispiel 1

**Elektrochemische Fluorierung von 3-n-Butoxy-tetrahydrothiophen-1,1-dioxid**

Nach DE-PS 682 079 wird das Additonsprodukt aus Δ 3-Dihydrothiophen-1,1-dioxid und n-Butanol hergestellt. Das resultierende 3-n-Butoxy-tetrahydrothiophen-1,1-dioxid siedet bei 123-130°C und 10 mbar.

Dieses wird in einer in DE-OS 2 715 211 näher beschriebenen Apparatur in einer anfänglichen Konzentration von 2 Gewichtsprozent in Fluorwasserstoff gelöst und bei einer Stromdichte von 4 - 20 $mA/cm^2$ Anodenfläche über 35 Tage elektrolysiert. Fluorwasserstoff wird je nach Verbrauch, 3-n-Butoxy-tetrahydrothiophen-1,1-dioxid je nach Stromaufnahme nachdosiert. Von Zeit zu Zeit wird das Perfluorrohprodukt aus dem Elektrolytsystem abgelassen. Insgesamt werden 27,9 kg Ausgangsprodukt eingesetzt und 40,6 kg Perfluorrohprodukt gewonnen.

Eine Fraktionierung des Perfluorrohproduktes liefert 23,3 kg eines 1 : 4 Gemisches, das aus 4-Oxa-3-trifluormethyl-perfluoroctyl-1-sulfofluorid sowie 3-Oxa-2-pentafluorethyl-perfluorheptyl-1-sulfofluorid besteht.

Die Ausbeute bezogen auf eingesetztes 3-n-Butoxy-tetrahydrothiophen-1,1-dioxid beträgt 31 Prozent.

Beispiel 2

Elektrochemische Fluorierung von 3-Isopropoxy-tetrahydrothiophen-1,1-dioxid

Nach DE-PS 682 079 wird 3-Isopropoxy-tetrahydrothiophen-1,1-dioxid synthetisiert und analog Beispiel 1 elektrolysiert. Bei Einsatz von 30 kg der Ausgangsverbindungen erhält man 28,4 kg eines 1 : 4 Gemisches an 4-Oxa-3,5-bis-trifluormethyl-perfluorhexyl-1-sulfofluorid und 3-Oxa-2-pentafluorethyl-4-trifluormethyl-perfluorpentyl-1-sulfofluorid. Dies entspricht einer Ausbeute von 36 Prozent bezogen auf eingesetztes Ausgangsprodukt.

Beispiel 3

Elektrochemische Fluorierung von 3-Methoxy-tetrahydrothiophen-1,1-dioxid

Die Herstellung von 3-Methoxy-tetrahydrothiophen-1,1-dioxid erfolgt gemäß DE-PS 682 079. Die Verbindung wird analog Beispiel 1 elektrochemisch fluoriert. 11 kg Ausgangsprodukt liefern 11,6 kg eines 1 : 4 Substanzgemisches an 4-Oxa-3-trifluormethyl-perfluorpentyl-1-sulfofluorid und 3-Oxa-2-pentafluorethyl-perfluorbutyl-1-sulfofluorid. Dies entspricht einer Ausbeute von 44 %.

Beispiel 4

Elektrochemische Fluorierung von 3-(2-Methoxy)-ethoxy-tetrahydrothiophen-1,1-dioxid

Die Ausgangsverbindung wird nach DE-PS 682 079 hergestellt und analog Beispiel 1 elektrofluoriert. 16 kg des Ausgangsproduktes liefern 11,2 kg eines 1 : 4 Gemisches 4,7-Dioxa-3-trifluormethyl-perfluoroctyl-1-sulfofluorid und 3,6-Dioxa-2-pentafluorethyl-perfluorheptyl-1-sulfofluorid. Dies entspricht einer Ausbeute von 28 Prozent.

Beispiel 5

Elektrochemische Fluorierung von 3-Cyclohexoxy-tetrahydrothiophen-1,1-dioxid

Nach DE-PS 682 079 wird 3-Cyclohexoxy-tetrahydrothiophen-1,1-dioxid hergestellt und analog Beispiel 1 elektrochemisch fluoriert. 14 kg des Ausgangsproduktes liefern 8,2 kg eines 1 : 4 Gemisches an 4-Oxa-3-trifluormethyl-4-perfluorcyclohexyl-perfluorbutyl-1-sulfofluorid und 3-Oxa-2-pentafluorethyl-3-perfluorcyclohexyl-perfluorpropyl-1-sulfofluorid. Die Ausbeute bezogen auf eingesetztes Produkt beträgt 22 Prozent.

Patentansprüche

1) Perfluorierte Sulfonsäurefluoride der allgemeinen Formel

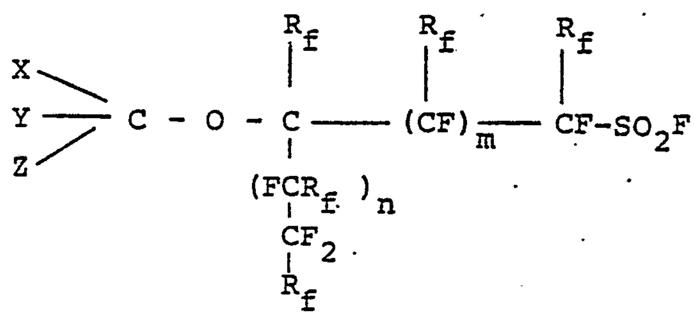

(I),

wobei

X, Y und Z unabhängig voneinander für F, einen Perfluoralkylrest mit 1 bis 8 C-Atomen in dem gegebenenfalls eine oder mehrere $CF_2$-Gruppen durch Sauerstoff ersetzt sein können, wobei die Sauerstoffatome durch mindestens eine $CF_2$-Gruppe voneinander getrennt sind, einem gegebenenfalls perfluoralkylsubstituierten Cycloperfluoralkylrest mit 5 - 12 C-Atomen, in dem eine oder mehrere $CF_2$-Gruppen durch Sauerstoff ersetzt sein können,

X und Y gemeinsam für einen gegebenenfalls perfluoralkylsubstituierten Cycloperfluoralkylrest, in dem eine oder mehrere $CF_2$-Gruppen durch Sauerstoff sein könne, $R_f$ unabhängig für F oder einen Perfluoralkylrest mit 1 - 3 C-Atomen und n sowie m für die Zahlen 0 oder 1 stehen.

2) Verfahren zur Herstellung perfluorierter Sulfonsäurefluoride gemäß Anspruch 1, dadurch gekennzeichnet, daß man Verbindungen der allgemeinen Formel

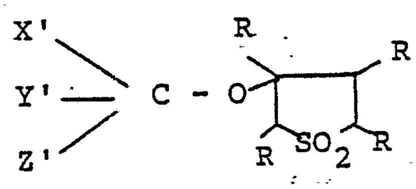

II,

in der X', Y' und Z' unabhängig voneinander für H, einen Alkylrest mit 1 - 8 C-Atomen, in dem eine oder mehrere $CH_2$-Gruppen durch Sauerstoff substituiert sein können, wobei die Sauerstoffatome durch mindestens eine $CH_2$-Gruppe voneinander getrennt sind, einen gegebenenfalls alkylsubstituierten Cycloalkylrest mit 5 - 12 C-Atomen, in dem eine oder mehrere der $CH_2$-Gruppen durch Sauerstoff ersetzt sein können,

X' und Y' gemeinsam für einen gegebenenfalls alkylsubstituierten Cycloalkylrest, in dem eine oder mehrere $CH_2$-Gruppen durch Sauerstoff substituiert sein können, R unabhängig für H oder einen Alkylrest mit 1 bis 3 C-Atomen stehen, der elektrochemischen Fluorierung unterwirft.

3) Verfahren nach Anspruch 2, dadurch gekennzeichnet, daß die Elektrofluorierung bei Temperaturen zwischen etwa -20°C und etwa 50°C durchgeführt wird.

4) Verfahren nach einem der Ansprüche 2 oder 3, dadurch gekennzeichnet, daß die Elektrofluorierung bei einer Stromdichte von 4 bis 20 $mA/cm^2$ wirksamer Anodenfläche durchgeführt wird.

5) Verwendung von perfluorierten Sulfonsäurefluoriden gemäß Anspruch 1 zur Herstellung oberflächenaktiver Substanzen.

6) Verwendung von perfluorierten Sulfonsäurefluoriden gemäß Anspruch 1 zur Herstellung von Ausgangsverbindungen zur hydro- und oleophoben Ausrüstung von Oberflächen.

**0052319**

Nummer der Anmeldung

Europäisches Patentamt

# EUROPÄISCHER RECHERCHENBERICHT

EP 81 10 9563.7

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.3) |
|---|---|---|---|
| A | DE - A1 - 2 735 210 (MONTEDISON)<br>* Anspruch 1; Seite 6, Zeilen 7 bis 9 *<br>-- | 1,5 | C 07 C 143/70<br>C 25 B 3/08<br>B 01 F 17/00 |
| A | DE - B2 - 2 201 649 (BAYER)<br>* Anspruch; Spalte 1, Zeilen 26 bis 32; Beispiele *<br>-- | 2,3,4,5 | |
| A | DE - A1 - 2 725 213 (BAYER)<br>* Seite 2, Zeilen 8 bis 12; Beispiel 1 *<br>---- | 3,4,5,6 | |

**RECHERCHIERTE SACHGEBIETE (Int. Cl.3)**

B 01 F 17/00
C 07 C 143/08
C 07 C 143/11
C 07 C 143/70
C 25 B 3/08

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

√ Der vorliegende Recherchenbericht wurde für alle Patentansprüche erstellt.

| Recherchenort | Abschlußdatum der Recherche | Prüfer |
|---|---|---|
| Berlin | 28-01-1982 | KNAACK |